Europäisches Patentamt

European Patent Office

Office européen des brevets

(19)

(11) **EP 1 201 746 A1**

(12) **EUROPEAN PATENT APPLICATION**

(43) Date of publication:
**02.05.2002 Bulletin 2002/18**

(51) Int Cl.⁷: **C12M 1/36**, C12P 19/02, C12N 1/20

(21) Application number: **01125217.8**

(22) Date of filing: **24.10.2001**

| | |
|---|---|
| (84) Designated Contracting States:<br>**AT BE CH CY DE DK ES FI FR GB GR IE IT LI LU MC NL PT SE TR**<br>Designated Extension States:<br>**AL LT LV MK RO SI** | (72) Inventors:<br>• **Gruenberg, Mads**<br>  **79100 Freiburg (DE)**<br>• **Posten, Clemens**<br>  **76229 Karlsruhe (DE)**<br>• **Rueckel, Markus**<br>  **82319 Starnberg (DE)** |
| (30) Priority: **31.10.2000 EP 00123710** | |
| (71) Applicant: **Roche Vitamins AG**<br>**4070 Basel (CH)** | (74) Representative: **Heiroth, Ulrike Hildegard et al**<br>**124 Grenzacherstrasse**<br>**4070 Basle (CH)** |

(54) **Optimisation of fermentation processes**

(57)    A method of optimised performance of bioprocesses involving complex nutrient mixtures, wherein the supply of each nutrient is periodically and alternately stopped until the metabolic activity of the micro-organisms decreases by a preset percentage, whereupon new feed concentrations of the complex nutrients are calculated and adjusted by means of an optimisation routine.

EP 1 201 746 A1

**Description**

The invention relates to a method of optimization of a bioprocess involving a complex nutrient mixture.

In fermentation processes, i.e. conversion of substances by using micro-organisms (hereinafter called bioprocesses for short) complex nutrients are frequently used as additional nutrient source for micro-organisms. Complex nutrients are raw materials containing two or more substances necessary for or promoting the growth of micro-organisms. Examples are in particular natural raw materials such as Cornsteep powder or liquor, a waste product in the extraction of starch from maize or yeast extract, and also synthetic mixtures of individual substances. A special advantage of these complex nutrients is the wide range of individual substances, such as amino acids, proteins, vitamins, mineral salts or trace elements, which can be made available to the micro-organisms. This is an advantage for obtaining high growth rates as compared with the use of minimal chemically defined media.

There are, however, various problems in using complex natural nutrients in bioprocesses. Since the substances are usually natural, the quality of complex nutrients varies widely in dependence on the manufacturer and the batch. Also the natural composition of complex nutrients is not necessarily optimal with regard to the actual requirement of the micro-organisms. Some constituents are present in too small quantities and therefore have a limiting effect, whereas others are present in excess and are either wasted or even inhibiting. Furthermore the overall metabolism of the many different constituents in complex nutrients is very complicated and partly unknown. Various overlapping adaptation processes may cause violent fluctuations in the process, resulting in irregular productivity and yield from the bioprocess. In multi-stage production processes in particular this may be a serious problem because the subsequent process steps may be affected. Also non-optimum operating conditions increase costs. Once-for-all optimisation of the medium with regard to a few key substances cannot be regarded as sufficient because the properties of complex nutrients are variable and so is the system itself, represented by the metabolising micro-organisms.

A known means of optimization involves e.g. the complete factorial experimental design whereby in a statistical approach all possible combinations of independent variables are investigated in suitable condition. A model of the system is therefore necessary. Although an optimum is quickly reached in the case of many substances and concentrations under investigation, these methods are impracticable when the number of variables and conditions is larger, owing to the enormous number of experiments required. A more efficient optimisation strategy is to plan tests to allow for some of the factors by the "Response Surface" method, e.g. the Plackett-Burmann method (Greasham and Inamine in: Demain and Solomon (Eds), Manual for industrial microbiology and biotechnology, Washington: ASM 1986, pages 41 - 48) or the Box-Behnken method (Greasham and Herber in: Rhodes and Stanbury (Eds), Applied microbiol physiology - a practical approach. Oxford: Oxford University Press 1997, pages 53 - 74). In these methods the number of variables is reduced to those with a significant effect, e.g. on growth or product formation.

Genetic algorithms, in contrast to statistical methods, are non-model-based methods of optimisation. With regard to application thereof, this means that they generally need not be based on theoretical considerations regarding the metabolism of micro-organisms. These methods can optimise a large number of media components in convergent manner. Out of a number of parallel shake flask experiments the best are selected, and the media therefrom become the starting points for the next generation of experiments. The procedure is repeated until convergence is reached. In the first generation the media are varied at random [Weuster-Botz et al., Biotechnol. Prog. 13:387 - 393 (1997)]. Weuster-Botz et al., Appl. Microbiol. Biotechnol. 46:209-219 (1996) optimised eight trace elements in 180 shake flask experiments by using a genetic algorithm, whereby the L-isoleucine concentration was improved by 50% compared with the standard medium. Weuster-Botz et al. (1996) used the same method in an L-lysine process to optimise 13 medium components in 472 standardised shake flask experiments. The L-lysine concentration was improved by over 2% as a result. Compared with statistical formulations including the "Response Surface" method with a conventional complete second-order polynomial model, the number of experiments was appreciably reduced - 472 instead of $2^{13}$ = 8192; all possible combinations of these parameters would have involved $101^{13}$ experiments. However there are serious disadvantages in medium optimisation by means of batchwise shake flask experiments. Usually the pH cannot be kept constant. The oxygen feed is very poor owing to the surface gas admission, and also, because of the variation in starter cultures, reproducibility is not always possible.

The pulse method in chemostatic culture [Kuhn et al., Eur. J. Appl. Microbiol. 6:341-349 (1979); Goldberg and Er-el, Proc. Biochem. 16:2-81 (1981); Fiechter, Adv. Biochem. Eng. Biotechnol. 30:7-60 (1984); Reiling et al., J. Biotechnol. 2:191-206 (1985)] uses a pulse injection technique to obtain growth reactions on nutrients. This is a means of identifying essential nutrients, the yield coefficients of which can be subsequently determined in a number of chemostatic experiments, in each of which an essential nutrient is the limiting factor. The yield coefficients can then be used to obtain an optimised balanced medium. Since however the essential nutrients must first be identified, the experimental work is considerable.

The known optimisation methods are unsatisfactory. The object of the invention is therefore to provide a method for optimum performance of bioprocesses using complex nutrients, wherein the proportion of complex nutrients in the medium during the process is constantly re-adapted to the actual requirement of the micro-organisms and the actual quality of the raw materials.

According to the invention it has been found that bioprocesses using complex nutrient mixtures can be optimised if the supply of each nutrient is periodically and alternately stopped until the metabolic activity of the micro-organisms decreases by a preset percentage. The time taken on each occasion is used as a response signal whereby new feed concentrations of the complex nutrients are calculated and adjusted by means of an optimisation routine. The waiting time between these negative pulses should be between ¼ and 1 hydrodynamic residence time, depending on the dynamics of the process. In some cases, however, the waiting time may be zero or even longer than 5 hydrodynamic residence times. The hydrodynamic residence time is the ratio of the flow rate (litres per hour) to the reaction volume in litres.

In continuous operation of an ideally mixed stirred tank (such as the bioreactors used), theoretically a complete volume exchange in the reactor is never reached. As an approximation, however, in chemical reaction technology a continuous stirred tank reactor is considered quasi-steady after three hydrodynamic residence times, since it is then calculated that 95% of the volume has been exchanged. In bioprocess technology, however, this time is at least five hydrodynamic residence times, since during the exchange of volume the micro-organisms react to the changed environment and, thus, delay reaching a quasi-steady state. In the optimisation routine of the invention there is no need to wait for a quasi-steady state after every negative pulse and this is, therefore, an advantage over conventional pulse-response methods.

The method according to the invention, as compared with methods based on a positive pulse response wherein the growth rate of the micro-organisms is temporarily increased by a nutrient pulse, also has the advantage that the measured response times are not falsified by lag phases of the micro-organisms. A lag phase is the time taken by the micro-organisms to adapt to changed ambient conditions. It is characterised in that the microbial growth initially remains almost unchanged. In positive nutrient pulses, the measurable reaction to the pulsed nutrient is delayed in usually non-reproducible manner, thus falsifying the response time.

The metabolic activity can be measured via observable process parameters, such as the oxygen transfer rate or the carbon dioxide transfer rate. "Carbon dioxide transfer rate" in this connection means the amount of carbon dioxide migrating per unit time from the liquid phase (fermentation broth) to the gas phase (exhaust gas). It can be directly measured by exhaust-gas analysis. Since the quantity of carbon dioxide not detected by exhaust-gas analysis and leaving the reactor in dissolved form is usually negligible, the rate of formation of carbon dioxide can be equated with the measured carbon dioxide transfer rate for the purposes of the invention. Other parameters of use for controlling the process are e.g. the pH, the concentration of dissolved oxygen and the temperature. The percentage reduction in metabolic activity, measured via the change in the said process parameters, should be chosen at a relatively small value (e.g. 1 - 5%) so that the process is not driven into conditions where the main substrate (e.g. sorbitol in the example) is not completely converted.

In industrial implementation of the optimisation process according to the invention, preferably the ratio of the feed concentrations of the complex nutrients and the total quantity of the complex nutrients are regarded as separate control variables but are adjusted simultaneously.

According to the invention the proportions and the total quantity can be simultaneously adjusted by an optimisation routine centred preferably on a multi-component controller. The multi-component controller can e.g. be based on fuzzy logic [compare Zadeh, Inf. Control 8:338-353 (1965)]. The optimisation routine preferably comprises the following three levels:

    1. The co-ordination controller for generating the control variables;
    2. Multicomponent controllers (e.g. fuzzy-logic controllers) and
    3. Control of the feed concentrations of the complex nutrients.

"Optimisation routine" means an arrangement of elements which in co-operation can be used to control the process in the desired sense. An optimisation routine according to the invention may e.g. involve a co-ordination controller using the negative-pulse response technique, generating response times and using them to form the input variable $Q_{sens}$. In all cases the pulse response time is measured for one nutrient while the other is stopped. The reciprocal $Q'_{sens}$ is used as the input variable for the stopped nutrient. The co-ordination controller also calculates the input variable relative to the set value for adjusting the total quantity $\Psi_{GM}$. The multicomponent controller is run through once for each of the two nutrients ($c_{N1,F,I}$ and $c_{N2,F,I}$ = feed concentrations of the complex nutrients; in this expression the subscripts N1 and N2 denote the various complex nutrients, F denotes the feed concentration and I is a serial subscript within the optimisation routine.) The respective feed concentrations of the complex nutrients are then re-calculated via the controller output.

Control variable for optimising the quantitative proportions:

The negative pulses are completed for each complex nutrient while the other is stopped. In this case the control variable is the rate of formation of $CO_2$ (measured as the transfer rate).

For example, after the supply of one complex nutrient has been stopped, the time is measured before the rate of formation of carbon dioxide decreases by 3%. The optimisation algorithm then calculates new feed concentrations for the two complex nutrients. After a fixed waiting time (in this example 5 h corresponding to half the residence time), a negative pulse is completed for the other complex nutrient. Since this method is a convergence method, it is not necessary to reach a steady state after each negative pulse.

The relevant control variable for optimising the quantitative proportions:

$$Q_{sens} = \frac{\Delta t_i}{\Delta t_{i-1}} \tag{1}$$

is therefore obtained by dividing the actual pulse response time $\Delta t_i$ by the pulse response time $\Delta t_{i-1}$ in the previous cycle, measured with the respective other complex nutrient. In the case of the stopped nutrient, the reciprocal of $Q_{sens}$

$$Q'_{sens} = \frac{\Delta t_{i-1}}{\Delta t_i} \tag{2}$$

is used. The same fuzzy-logic controller can therefore then be used, thus appreciably reducing the efforts in tuning the controller. Owing to the hydrodynamic properties of the stirred tank reactor, the response times and consequently the denominators in (1) and (2) cannot become zero.

The control variable for the total quantity

The control variable for the total quantity is the value of a parameter $x_{GM}$ observable during the process and correlated, e.g. with the biomass or with the yield of product, e.g. the rate of oxygen consumption or the virtual concentration of carbon dioxide. For inputting into the fuzzy-logic controller, the control variable is standardised at the set point, yielding the following general input variable:

$$\Psi_{GM} = \frac{X_{GM}}{X_{GM.soll}} = \frac{\text{control variable}}{\text{set point}} \tag{3}$$

Extension to more than two complex nutrients:

When there are two complex nutrients for optimising, the result is a cycle in two different steps. In principle however the loop can be extended to any number of steps, i.e. complex nutrients. In the generalised case of n complex nutrients, we can write

$$Q_{sens,i} = \frac{(n-1)\,\Delta t_i}{\sum\limits_{q=i-n}^{i-1} \Delta t_q} \tag{4}$$

This does not change the parameter $\Psi_{GM}$ for controlling the total quantity. Owing to the long time constants, it is not a practical proposition to optimise more than three or four nutrients.

Controlling the feed concentration:

Corresponding to the output $x_a$ of the multicomponent controller, the feed concentrations of the complex nutrients are controlled as follows:

$$c_{k,F,i+1} = x_a \bullet c_{k,F,i}, \ k = N1, N2 \tag{5}$$

The process of the invention is applicable to all fermentation processes involving complex nutrients, e.g. the conversion of D-sorbitol to L-sorbose using a microorganism. The microorganism may be any microorganism useful for the respective conversion, e.g. a *Gluconobacter suboxydans* strain may be used for the conversion of D-sorbitol to L-sorbose, e.g. *G. suboxydans* IFO 3291 which was deposited with the Institute for Fermentation, Osaka, Japan on April 5, 1954, or which was deposited as a mixed culture with *G. oxydans* DSM 4025 under the Budapest Treaty as FERM BP-3813 at the Fermentation Research Institute, Japan, on March 30, 1992.

For the purpose of continuous cultivation of the microorganism, e.g. *G. suboxydans*, and in order to implement the desired optimisation process, the fermentation system comprises

1. a bioreactor equipped for continuous operation;

2. a means for separating the feed of medium into a number of streams of the individual components, so that the composition of the medium can be altered during the process;

3. a means for measurement and control of pH, $pO_2$ and temperature;

4. a device for measuring and controlling the filling level of the bioreactor to ensure efficient and continuous operation;

5. a means for controlling the feed stream and measuring the exhaust-gas composition, so that corresponding gas transfer rates are available as measurement signals, and

6. an automation system for controlling the bioprocess installation.

The bioreactor may e.g. be a standard, e.g., laboratory bioreactor with suitable additional equipment and an automation system, e.g. in an automated, e.g., laboratory system storage bottles and the bottle for caustic soda solution, the control unit of the bioreactor, the bioreactor itself together with measuring probes and the product container, lines for gas admission together with the mass flow controller and sterile filter and the $CO_2$ and $O_2$ analysis for the exhaust gas, the process computer and the serial interfaces, electric wires for data transmission, the corresponding form of transmission being, e.g. RS-232, RS-422 or Mettler Local-CAN.

The invention will now be further explained with reference to an exemplified conversion of D-sorbitol to L-sorbose using *G. suboxydans*:

Example: Continuous cultivation of *Gluconobacter suboxydans*, wherein D-sorbitol is converted to L-sorbose.

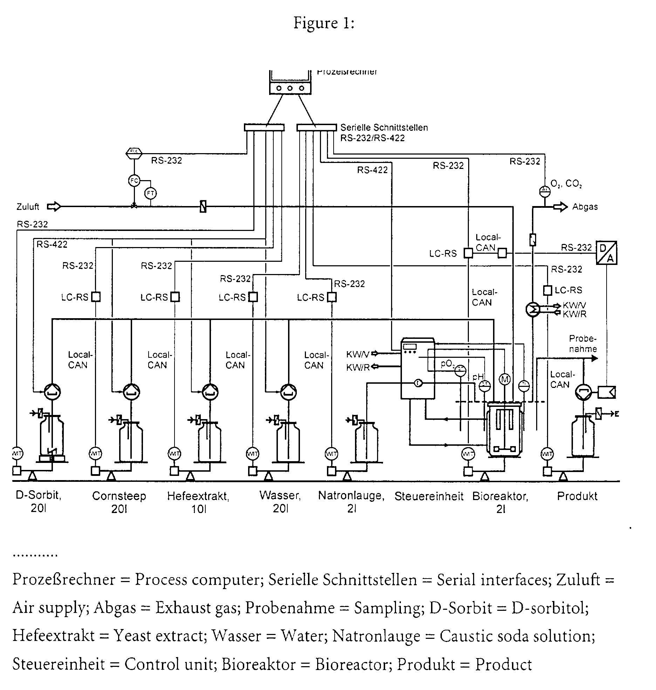

For the fermentation a standard laboratory bioreactor with additional equipment and automation system components according to Figure 1 was used.

Figure 1: Automated laboratory system; the bottom row shows the four storage bottles (left) and the bottle for caustic soda solution. To the right are the control unit of the bioreactor, the bioreactor itself together with measuring probes and the product container. The lines for gas admission together with the mass flow controller and sterile filter and the $CO_2$ and $O_2$ analysis for the exhaust gas are represented under the process computer and the serial interfaces. The thin lines indicate the electric wires for data transmission, showing the corresponding form of transmission (RS-232, RS-422 or Mettler Local-CAN).

The process computer was a commercial Server-PC. The equipment chosen for the process computer was as follows: Server-PC'Dell PowerEdge 2200'; 2 Intel Pentium II 300 MHz CPUs; 128 MB main memory, 2 graphic cards and 2 screens (21"); 2 Control RocketPort 16 ISA multiport serial cards for a total of 32 serial interfaces, each switchable between RS-232 and RS-422

Software: The operating system of the process computer was Microsoft Windows NT 4.0 (Service Pack 3).

Automation was based on the industrial software BridgeVIEW, Version 1.1, by National Instruments.

Fuzzy logic was applied by means of the BridgeVIEW extension DataEngine VI 1.5 by MIT GmbH, Aachen.

The Bioreactor: The bioreactor was a standard Biostat B standard stirred tank reactor with a working volume of 2 litres by B. Braun Biotech International. The inlet air was introduced into the bioreactor through a silicon flexible tube and a sterile filter (pore size 0.2 $\mu$m). The gas introduction means was a gasification ring, also supplied, disposed underneath the 6-blade disc stirrer. The exhaust gas was first conveyed through a condenser on the bioreactor and then through a flexible silicon tube and a sterile filter (pore size 0.2 $\mu$m) to the exhaust-gas analysers. The bioreactors were each equipped with a pH electrode and $pO_2$ probe (both by Ingold) and a temperature probe (PT100). The bioreactors were equipped in the factory with a control unit containing the measurement amplifiers for pH, $pO_2$ and temperature probes and the initially required standard controllers for these parameters.

The transfer of process data and set values between the controller and the process computer occurred via a serial RS-422 interface. The pH electrode was calibrated (two-point calibration at pH = 7.00 and pH = 4.01) before each sterilisation operation. The $pO_2$ probe was calibrated after sterilisation (single-point calibration with 100% air saturation in the medium).

Control of filling level of the bioreactor: The filling level of the bioreactor was controlled via the weight. A balance (Mettler Toledo SG32001) was disposed under the reactors and yielded digital signals (serial RS-232 interface) which were converted to a 4-20 mA signal in a digital/analog converter. The analog signal was connected to the input of a hardware controller (Eurotherm), which actuated the discharge pump (Gilson Minipuls 3 peristaltic pump) of the bioreactor, using an analog 0-10 V signal.

Storage solutions: Five different storage solutions were used to make up the medium, and were each added separately.

The mass flow measurement was gravimetric. The signals from the balance were transmitted via serial RS-232 interfaces to the process computer. The various balances used are listed in Table 1. LC-RS adapters by Mettler-Toledo were used for the type SG and PG balances.

To prevent the formation of density gradients, the D-sorbitol storage bottle was stirred by a magnetic stirrer (produced by Variomag) disposed between the balance and the bottle.

The media were conveyed by peristaltic pumps (Gilson MiniPuls 3) through flexible silicon tubes to the bioreactor. For communication to the peristaltic pumps a serial RS-422 bus was used. To this bus up to ten pumps could be connected. Since the pumps had a so-called GSIOC interface, a suitable adapter was used on each RS-422 bus.

Table 1:

| No. | Contents | Type of balance | ML | Accuracy |
|---|---|---|---|---|
| \multicolumn{5}{} Balances used [Mettler-Toledo] and maximum load [ML] |
| 1 | D-sorbitol | KCC 150s with ID 5 | 150 kg | 1 g |
| 2 | Cornsteep | SG32001 DR | 32 kg | 0.1 g |
| 3 | Yeast extract | SG32001 DR | 32 kg | 0.1 g |
| 4 | Water | SG32001 DR | 32 kg | 0.1 g |
| 5 | Caustic soda solution | PG8002 | 8 kg | 0.01 g |

Inlet air control: The inlet air streams were controlled by gas mass flow controllers type 1179 by MKS, Munich, operating on the principle of the hot-wire anemometer. The power supply and analog control and evaluation of the gas mass flow controller were effected via a type 647B 4-channel control device by MKS, connected to the process computer via RS-232. Since the measurements by the controller were based on the thermal capacity of the gas being measured, suitable gas corrective factors were set up. The mass flow of gas was expressed in standard volumes per unit time ($Ncm^3$ $min^{-1}$, standard conditions, T = 273.14 K; p = 0.101325 MPa). The measuring range was 2000 $Ncm^3$ $min^{-1}$ at an accuracy of 1.0% of the maximal range. The measured mass flow of gas was calibrated at the factory for nitrogen. For air the gas corrective factor was 1.0.

Exhaust-gas analysers: The exhaust-gas analyser comprised a microprocessor-controlled oxygen analyser OXOR 610 and a microprocessor-controlled NDIR gas analyser (UNOR 610 by Maihak, Hamburg) for measuring carbon dioxide. Both devices were connected to the process computer via RS-232.

Sterilisation: The bioreactor, all the feed and discharge pipes and the vessels for products were sterilised for 20 minutes in a saturated steam atmosphere (0.2 MPa at 121°C). The sterile storage solutions and the air-feed and exhaust-gas lines were connected via special steel sterile couplings.

The micro-organism: The micro-organism *G. suboxydans*, IFO 3291 was used which was deposited under the Budapest Treaty as FERM BP-3813 at the Fermentation Research Institute, Japan on March 30, 1992.

Media: The continuous medium was made up of four separate storage solutions. For simple determination of the dry biomass, all the solutions had to be free from solids. Since Cornsteep powder contains a high proportion of insoluble constituents, the Cornsteep solutions were suitably processed (see hereinafter). The concentrations of the resulting media are given in g/$L^{-1}$. However, the individual storage solutions from which the resulting medium was made up, were reckoned in percentages by weight, to simplify production thereof by weighing in the individual components. The following solutions were used:

1. D-sorbitol solution, 50.4% D-sorbitol, ρ = 1.22 kg $l^{-1}$; lot size: 20 l
2. Cornsteep solution: 2% Cornsteep powder (Roquette, France) and salts as per Table 2 in demineralised water; batch size: 20 l. Before sterilisation the solution was centrifuged at 4000 g for 10 minutes. The sterilised solution was filtered into an empty sterile 20 l bottle through a 3 μm deep-bed filter module (Sartorius 5521307P900A, sterile) in front of a 0.2 μm membrane filter module (Gelman Supor DCF CFS92DS, sterile); ρ = 1.01 kg $l^{-1}$.
3. Yeast extract solution; 4% yeast extract powder, Oxoid, and salts as per Table 2 in demineralised water; ρ = 1.01 kg $l^{-1}$; batch size: 10 l
4. Water: salts as per Table 2 in demineralised water; ρ = 1.00 kg $l^{-1}$; batch size: 20 l
5. 3 N caustic soda solution for adjusting the pH; ρ = 1.25 kg $l^{-1}$; batch size: 2 l.

Since the micro-organism used always produces small quantities of acid metabolites, no acid was needed for adjusting the pH. The concentrations of the solutions of complex nutrients relate to the corresponding dry powder as weighed in. In the case of the Cornsteep solution this means that the separated solids are also contained in the stated concentration. Since however solids are not usually bioavailable, comparison may be made with Cornsteep solution containing solids as used on the pilot or production scale.

Table 2:

| Concentrations of salt in the storage solutions 2 to 4 [A] and in the resulting medium [B] | | | |
|---|---|---|---|
| Salt | A at $c_{sit.F}$ = 275 g/l/% | A at $c_{sit,F}$ =137.5 g/l/% | B/(g l⁻¹) |
| $MgCl_2 \cdot 6\ H_2O$ | 0.029 | 0.021 | 0.176 |
| $KH_2PO_4$ | 0.055 | 0.039 | 0.330 |
| $CaCl_2 \cdot 2\ H_2O$ | 0.014 | 0.010 | 0.083 |

The salt concentration in the resulting medium should be similar to that of synthetic water. The salt concentrations in solutions 2 and 4 and in the medium resulting from the first four solutions can be obtained from Table 2. Since the D-sorbitol solution (solution 1) did not contain any salts, the salt concentrations of solutions 2 to 4 must be correspondingly higher. The consumption of solution 5 was negligible and was disregarded when calculating the salt concentrations.

All the solutions were sterilised for 20 minutes in a saturated steam atmosphere (0.2 MPa at 121°C).

A constant dilution rate of D = 0.1 h⁻¹ and a constant D-sorbitol concentration of $c_{sit.F}$ = 275 g l⁻¹ in the feed were chosen. The feed concentrations of Cornsteep and yeast extract were preset by the optimisation process. On each occasion the bioprocess automation system, using the preset concentrations and the dilution rate, calculated the required mass flows for each storage solution. The calculated mass flows were converted and kept constant in the controller incorporated in the bioprocess automation system. The cycle time was 1 second. This ensured that the negative pulses generated by the optimisation routine and the newly calculated feed concentrations of the complex nutrients were exactly adhered to.

Implementation of the optimisation routine for this special example: The chosen characteristic measurement signal for the metabolic activity was the carbon dioxide production rate CPR.

The characteristic measurement signal for the total amount was the virtual carbon dioxide concentration (D-sorbitol equivalent per volume)

$$c_{CO2,virt} = \frac{CPR \cdot M_{Sorbitol}}{6 \cdot 22.4\ l/mol^{-1} \cdot D} \qquad (6)$$

Since the dilution rate D in general was constant, the variation in the carbon dioxide production rate CPR and in virtual carbon dioxide concentrations $C_{CO2.virt}$ vary in linear manner.

In the special case in this example, therefore, the control variable for the total quantity is

$$\Psi_{GM} = \frac{c_{CO_2,virt}}{c_{CO_2,virt,soll}} \qquad (7)$$

calculated from the actual virtual carbon dioxide concentration and the set value for the virtual carbon dioxide concentration.

In this special example the multicomponent controller was constructed in the form of a fuzzy-logic controller with, e.g., the following fuzzy correlation functions: For example the numerical value 0.7 of the function "very low" is given a correlation of 0.33 and of the function "low" is given a correlation of 0.67. In other words the numerical value $\Psi_{GM}$ = 0.7 in the linguistic sense means 33% 'very low' and 67% 'low'.

The numerical values of the control variables $Q_{sens}$ and $\Psi_{GM}$ or $Q'_{sens}$ and $\Psi_{GM}$ are translated into fuzzy-logic linguistic variables containing so-called correspondence functions. This process is also called 'fuzzyfication'. Since the same fuzzy-logic controller, only with different control variables, was used for the stopped complex nutrient and for the complex nutrient whose pulse response time was measured during the actual cycle, no distinction hereinafter is made between $Q_{sens}$ and $Q'_{sens}$. The linguistic input variables $Q_{sens}$ and $\Psi_{GM}$ are linked and the linguistic output variables are associated by 'if......... then' rules. Since the association with the correlation functions is sharp, a number of rules may apply simultaneously.

The rules are weighted on the basis of the correlation values. The linguistic starting variable is transformed back to a numerical value representing the controller output $x_a$. This process is also called 'defuzzyfication'

Further information about fuzzy logic can be obtained from the relevant literature [Zimmermann (Ed): Fuzzy-Technologien - Prinzipien, Werkzeuge, Potentiale. Düsseldorf: VDI-Verlag (1993)].

Fermentation runs completed by using the optimisation routine: The adjusted process was observed over a period of 13 days. The control variable $c_{CO2.virt}$ and both correction variables fluctuate with a time offset of about half a day from one another. Apparently the fuzzy-logic controller reacts somewhat too sharply to deviations of the control variable $c_{co2.virt}$ from the set value. These fluctuations, however, do not increase and the process as a whole remains stable. The control variable $Q_{sens}$ fluctuates at irregular intervals around its set value. Even here, no unstable behaviour was observed.

Optimising the quantitative proportions: In order fully to stabilise the control variable $Q_{sens}$ artificial fluctuations were produced in the quality of the complex nutrients. To this end the storage bottles of the complex nutrients were respectively replaced by bottles containing complex nutrients from other manufacturers and therefore of different quality.

A specially informative example was the change form Oxoid yeast extract to Roth yeast extract. The ratio of Cornsteep to yeast extract altered from about 3:1 to 1:1. After another change back to Oxoid yeast extract, the old proportions were restored. The adjustment took about 3 days or 7 hydrodynamic residence times.

The change in the yeast extract results in some cases in considerable deviations from the set values. The optimisation process however can keep the process stable even in these situations.

Adjustment of the total quantity: Variation in time of the virtual carbon dioxide concentration $c_{co2.virt}$ and of the feed concentrations of the complex nutrients $c_{CS,F}$ and $c_{YE.F}$ after switching on the optimisation process with excursion of the control variable for the total quantity of complex nutrients $c_{co2.virt}$.adjustment of the virtual carbon dioxide concentration $c_{co2.virt}$ from a relatively high starting value of about 13 g l$^{-1}$ to the set value of 6 g l$^{-1}$. An overshoot is clearly recognisable in the right-hand of part 7. The adjustment took 4 days or about 10 residence times.

After reaching the set values the molar yield with regard to L-sorbose improved from 90.3% to 91.1%. The concentrations of complex nutrients $c_{CS,F}$ and $c_{YE,F}$ in the feed were reduced on average by 51% and 56% respectively.

**Claims**

1. A method of optimised performance of bioprocesses involving complex nutrient mixtures, wherein the supply of each nutrient is periodically and alternately stopped until the metabolic activity of the micro-organisms decreases by a preset percentage, whereupon new feed concentrations of the complex nutrients are calculated and adjusted by means of an optimisation routine.

2. A method according to claim 1, wherein the optimisation routine comprises a co-ordination controller for generating the control variables, a multicomponent controller and a means for controlling the feed concentrations of the complex nutrients.

3. A method according to claims 1 and 2, wherein two different complex nutrient mixtures are used.

4. A method according to claims 1 or 2, wherein the optimisation routine corresponds to a flow chart involving a co-ordination controller using the negative-pulse response technique, generating response times and using them to form the input variable $Q_{sens}$.

5. A method according to claims 2 to 4, wherein the multicomponent controller is a fuzzy-logic controller.

6. A method according to claims 1 to 5, wherein the ratio between the feed concentrations of the complex nutrients and the total quantity of the complex nutrients are considered as separate control variables but are adjusted simultaneously.

7. A method according to claims 1 to 6, wherein the micro-organism is *Gluconobacter suboxydans.*

8. A method according to claim 7, wherein D-sorbitol is converted to L-sorbose.

9. A device for optimised performance of micro-biological processes involving complex nutrient mixtures, wherein the supply of each nutrient is periodically and alternately stopped until the metabolic activity of the micro--organisms decreases by a preset percentage, whereupon new feed concentrations of the complex nutrients are calculated and adjusted by means of an optimisation routine, the device comprising

    a) a reactor for performing the micro-biological process and comprising at least two individual feed lines for supplying nutrients;
b) sensors for measuring the metabolic activity of the micro-organisms;
c) a co-ordination controller controlled by the sensors;
d) a multicomponent controller and
e) elements for controlling the feed concentrations of the complex nutrients.

**10.** A device according to claim 9, wherein the elements b) to e) are disposed as in Fig. 1.

**11.** The invention as hereinbefore described, particularly in the examples and drawings.

Figure 1:

...........

Prozeßrechner = Process computer; Serielle Schnittstellen = Serial interfaces; Zuluft =
Air supply; Abgas = Exhaust gas; Probenahme = Sampling; D-Sorbit = D-sorbitol;
Hefeextrakt = Yeast extract; Wasser = Water; Natronlauge = Caustic soda solution;
Steuereinheit = Control unit; Bioreaktor = Bioreactor; Produkt = Product

**European Patent Office**

## EUROPEAN SEARCH REPORT

Application Number

### DOCUMENTS CONSIDERED TO BE RELEVANT

| Category | Citation of document with indication, where appropriate, of relevant passages | Relevant to claim | CLASSIFICATION OF THE APPLICATION (Int.Cl.7) |
|---|---|---|---|
| A | DE WULF PETER ET AL: "Optimized synthesis of L-sorbose by C5-dehydrogenation of D-sorbitol with Gluconobacter oxydans." BIOTECHNOLOGY AND BIOENGINEERING, vol. 69, no. 3, 4 August 2000 (2000-08-04), pages 339-343, XP002188371 ISSN: 0006-3592 * the whole document * | 7,8 | C12M1/36 C12P19/02 C12N1/20 |
| A | FR 2 771 421 A (BERTIN & CIE) 28 May 1999 (1999-05-28) * claim 11; figures 1,2 * | 9 | |
| A | THATIPAMALA RAMAKRISHNAIAH ET AL: "On-line state estimation and adaptive optimization using state equations for continuous production of bioethanol." JOURNAL OF BIOTECHNOLOGY, vol. 48, no. 3, 1996, pages 179-190, XP004037015 ISSN: 0168-1656 * the whole document * | | TECHNICAL FIELDS SEARCHED (Int.Cl.7) C12N C12M C12P |
| A | WO 97 36993 A (PRIVATE UNI WITTEN HERDECKE GM ;BARTHOLMES PETER (DE); ROSENTHAL W) 9 October 1997 (1997-10-09) * the whole document * | | |

The present search report has been drawn up for all claims

| Place of search | Date of completion of the search | Examiner |
|---|---|---|
| THE HAGUE | 25 January 2002 | Lejeune, R |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons

& : member of the same patent family, corresponding document

EPO FORM 1503 03.82 (P04C01)

**ANNEX TO THE EUROPEAN SEARCH REPORT
ON EUROPEAN PATENT APPLICATION NO.**   EP 01 12 5217

This annex lists the patent family members relating to the patent documents cited in the above-mentioned European search report.
The members are as contained in the European Patent Office EDP file on
The European Patent Office is in no way liable for these particulars which are merely given for the purpose of information.

25-01-2002

| Patent document cited in search report | | Publication date | Patent family member(s) | | Publication date |
|---|---|---|---|---|---|
| FR 2771421 | A | 28-05-1999 | FR | 2771421 A1 | 28-05-1999 |
| | | | WO | 9928438 A1 | 10-06-1999 |
| WO 9736993 | A | 09-10-1997 | DE | 19612766 A1 | 02-10-1997 |
| | | | AT | 197473 T | 11-11-2000 |
| | | | DE | 59702606 D1 | 14-12-2000 |
| | | | DK | 889950 T3 | 27-12-2000 |
| | | | WO | 9736993 A1 | 09-10-1997 |
| | | | EP | 0889950 A1 | 13-01-1999 |

EPO FORM P0459